# EUROPEAN PATENT APPLICATION

(11) **EP 4 029 477 A1**
(43) Date of publication of application: **20.07.2022**
(21) Application number: 21839307.2
(22) Date of filing: 18.01.2021
(51) Int. Cl.: A61F 2/24

(54) **MITRAL VALVE DEVICE IMPLANTED VIA ATRIAL SEPTUM, AND IMPLANTATION METHOD**

(30) Priority: 23.11.2020 CN 202011320550; 23.11.2020 CN 202022725383 U
(71) Applicant: Jiangsu Trulive Medtech Co., Ltd, Rudong County Nantong Jiangsu 226400 (CN)
(72) Inventor: ZHAO, Jing, Shanghai 201206 (CN)
(74) Representative: Argyma
(86) International application number: PCT/CN2021/072489
(87) International publication number: WO 2022/105054

(57) **Abstract**

A transseptal mitral valve apparatus is provided. The transseptal mitral valve apparatus includes a valve prosthesis and a ventricle anchoring member. The valve prosthesis includes a stent body playing a supporting role and a prosthetic valve leaflet. The prosthetic valve leaflet is fixed at a pre-set position of the stent body. The ventricle anchoring member includes a connecting portion and an anchoring portion attached to a ventricle inner all. The anchoring portion is fixed to the stent body by the connecting portion. The invention further provides a method for implanting the transseptal mitral valve apparatus.

## Description

### BACKGROUND OF THE INVENTION

### Field of the Invention

The invention relates to the technical field of medical instruments, in particular, to a transseptal mitral valve apparatus for replacing a native valve.

### Description of the Prior Art

The heart contains four cavities, i.e., right atrium (RA), right ventricle (RV), left atrium (LA), and left ventricle (LV). During the whole cardiac cycle, pumping on the left and right sides of the heart usually occurs synchronously. A valve dividing the atrium from the ventricle is called an atrioventricular valve. The atrioventricular valve performs a "one-way valve" function for ensuring the normal flow of blood in the cardiac cavity. The atrioventricular valve between the left atrium and the left ventricle is mitral valve, and the atrioventricular valve between the right atrium and the right ventricle is tricuspid valve. Pulmonary valve directs the blood flow to a pulmonary artery and then the blood from the pulmonary artery to the lung; afterward, the blood returns to the left atrium through the pulmonary vein. Aortic valve directs the blood flow through an aorta and from there to the surrounding regions. Normally, there is no direct connection between the atriums or the ventricles.

When the ventricle begins to fill (diastole), the aortic valve and the pulmonary valve close for preventing a regurgitation from the aorta to the ventricle. Shortly thereafter, the atrioventricular valve opens to allow unobstructed flow from the atrium into the corresponding ventricle. Shortly after the ventricular systole (i.e., the ventricular emptying) begins, the tricuspid valve and the mitral valve normally close, thereby forming a seal that prevents the regurgitation from the ventricle to the corresponding atrium.

When there is a problem with the atrioventricular valve, the atrioventricular valve cannot function properly, resulting in closing improperly. The atrioventricular valve is a complicated structure, usually including a valve annulus, valve leaflets, a chordae tendineae, and a support structure. Each of the atriums is connected to the valve thereof by an atrium vestibule. The mitral valve has two leaflets, and the attachment or engagement between the corresponding surfaces of the valve leaflets facilitates providing closure or sealing to the valve, thereby preventing the blood from flowing in an incorrect direction. During the ventricular systole, a situation where the valve leaflets cannot be sealed is called a poor engagement, which causes the blood to flow reversely through the valve (i.e., the regurgitation). The heart valve regurgitation may cause severe results for the patients, typically resulting in heart failure, reduction of the blood flow amount, reduction of blood pressure, and/or reduction of oxygen flow amount reaching the human tissues. The mitral valve regurgitation may further cause the blood to flow from the left atrium back to the pulmonary vein, resulting in hyperemia. A severe closure insufficiency of valvular, if not treated, may cause permanent disability or death.

Transcatheter Mitral Valve Replacement (TMVR) is a method of using the intervention of catheters, wherein the prosthetic valve is compressed to the delivery system in vitro, sent to the mitral valve annulus of the human body along the blood vessel path or through the apex cordis, and then released and fixed to the mitral valve annulus for replacing the native valve. Compared with surgery, TMVR does not require extracorporeal circulation aids, causes small trauma, and allows the patients to recover quickly. Furthermore, the hemodynamic index of the patients improves significantly after the operation; meanwhile, compared with passing through the path of the apex cordis, the path passing through the interatrial septum via the femoral vein results in smaller trauma and is further widely accepted.

Although the technique of mitral valve replacement develops rapidly, there are still some recognized challenges in the design of the valve, e.g., anchoring for the valve. Some existing designs for the mitral valve basically perform anchoring by clamping the valve leaflets or capturing the valve leaflets. These two anchoring ways stretch the chordae tendineae, causing damages on the native valve leaflets. Some other designs perform the anchoring by the Oversize design of the stent body. With this anchoring way, the stent compresses the tissue, affects the heart contraction, and carries the risk of conduction block. And also, some designs perform the anchoring by tethers from the apex cordis. However, this anchoring way cannot realize the way of implanting via the interatrial septum path.

### SUMMARY OF THE INVENTION

The present invention provides a transseptal mitral valve apparatus for solving the above-mentioned drawbacks in the prior art.

The technical solution of the invention is as follows:
a transseptal mitral valve apparatus includes:
   a valve prosthesis, including a stent body playing a supporting role and a prosthetic valve leaflet, the prosthetic valve leaflet being fixed to the stent body; and
   a ventricle anchoring member, including a connecting portion and an anchoring portion attached into a ventricle inner wall, the anchoring portion being fixed to the stent body by the connecting portion;
after the valve apparatus is released, the valve prosthesis is fixed at a native valve annulus for replacing a native valve to play a role in opening or closing a blood flow channel, and the ventricle anchoring member provides a firm anchoring force to the stent body by the attachment between the anchoring portion and the ventricle wall so that the stent body is prevented from being impacted into a left atrium.

In an embodiment, the anchoring portion includes an anchoring body, and the anchoring body is configured to be of a mesh structure with a pre-set size and a pre-set shape. The anchoring portion is in a compressed state when being loaded into a delivery system, and is released and expanded to be of the above mesh structure with a pre-set size and shape after being implanted into a target position. The mesh structure facilitates loading, delivery and releasing the anchoring portion, and facilitates enlarging the contact area between the anchoring portion and the tissue to provide more forced points, so that not a single point is forced between the anchoring portion and the tissue; therefore, the force may be dispersed and forces will not be concentrated so that the tissue may not be damaged.

In an embodiment, the anchoring body is configured to be in a shape suitable for an inner wall of an apex cordis to be attached to the inner wall of an apex cordis. The tissue of the inner wall of the apex cordis is thicker, so that there is no horizontal force as compared to being anchored to a sidewall. Therefore, it is not easy to twist the stent body, so it is not easy to cause tear damages to the tissue when the anchoring portion is anchored to the tissue. Further, the region of the apex cordis has no chordae tendineae and papillary muscle and is relatively empty, so being anchored in the region of the apex cordis will not be disturbed by the chordae tendineae and the papillary muscle; in addition, compared to being released at the sidewall, being released at the inner wall of the apex cordis is performed in a linear path, and the releasing way is relatively easy.

In an embodiment, the anchoring portion further includes several anchoring apparatuses, the anchoring apparatuses are disposed on the anchoring body, and an enhanced anchoring effect is provided by the fixation between the anchoring apparatus and a tissue of a ventricle inner wall.

In an embodiment, the anchoring portion is provided with a retention portion, and the connecting portion is connected with the anchoring portion at the retention portion. Specifically, the retention portion may be provided with a retention hole located at a center of the anchoring portion, and the provision of the retention hole facilitates the stable connection between the connecting portion and the anchoring portion.

In an embodiment, the connecting portion is elastic-free, so as to provide a more stable anchoring force to the stent; or the connecting portion is elastic, so that a higher degree of stretch compliance is provided during the cardiac cycle.

In an embodiment, the anchoring portion is made by way of a weaving process or a cutting process.

In an embodiment, the anchoring portion is configured to be of an inverted cone structure, so that a cavity is formed between the anchoring portion and the ventricle wall to reduce a volume of a left ventricle after the ventricle anchoring member is released. The volume of the left ventricle is reduced by the cavity formed between the anchoring portion and the ventricle wall, so that a tension of the left ventricle of a patient is reduced, thereby playing a role in treating heart failure. The larger the left ventricle is, the better the therapeutic effect caused by reducing the volume of the left ventricle with the cavity is for the patient with poorer cardiac contractility.

In an embodiment, an end portion of the anchoring portion away from the stent body is provided with a force dissipating portion for increasing a contact area, such as being configured to be of a laminar structure and so on, so as to enlarge the contact area with the tissue.

In an embodiment, an elastic buffer portion is disposed between the force dissipating portion and the end portion of the anchoring portion, and the elastic buffer portion plays a buffering role. The force dissipating portion and the elastic buffer portion prevent the inner wall of the apex cordis from being damaged by the anchoring portion when the heart contracts.

In an embodiment, the anchoring portion is further covered with a skirt. The skirt may provide a buffering force and, in the long run, promote endothelialization, making the anchoring more stable. When the anchoring portion is configured to be of the inverted cone structure, the skirt may further play a sealing role, so as to isolate the above cavity.

The invention further provides a method for implanting a transseptal mitral valve apparatus, which includes providing a transseptal mitral valve apparatus, wherein the valve apparatus includes a valve prosthesis and a ventricle anchoring member, the valve prosthesis including a stent body playing a supporting role and a prosthetic valve leaflet, the prosthetic valve leaflet being fixed at a pre-set position of the stent body; the ventricle anchoring member includes a connecting portion and an anchoring portion attached to a ventricle inner all, the anchoring portion being fixed to the stent body by the connecting portion.

Compared with the conventional art, the present invention has the following beneficial effects:
First, the transseptal mitral valve apparatus of the invention may be implanted via the interatrial septum path, which causes less damages on the patient as compared being implanted via the apex cordis; in addition, according to the valve apparatus of the invention, the anchoring portion is released and then is attached to the ventricle inner wall to provide a firm anchoring force, which changes the traditional anchoring way of clamping the valve leaflets or capturing the valve leaflets, i.e., the valve apparatus of the invention will not stretch the chordae tendineae or damage the valve leaflets after being released, thereby reducing the damage on the tissue.
Second, according to the transseptal mitral valve apparatus of the invention, the anchoring body is configured to be of the mesh structure formed by arranging several mesh units with an enclosed shape; the anchoring portion is in a compressed state when being loaded into a delivery system, and is released and expanded to be of the above mesh structure with a pre-set size and shape after being implanted into a target position; the mesh structure facilitates loading, delivery and releasing the anchoring portion, and facilitates enlarging the contact area between the anchoring portion and the tissue to provide more forced points, so that not a single point is forced when the anchoring portion contacts the tissue; therefore, the force may be dispersed and forces will not be concentrated so that the tissue may not be damaged; further, the mesh structure makes the compliance of the anchoring portion better, and be adapted to the shape of the ventricle inner wall after the anchoring portion is released for being easier to form an anchoring relationship with the ventricle inner wall; meanwhile, the anchoring apparatus disposed on the anchoring body may provide the enhanced anchoring effect.
Third, according to the transseptal mitral valve apparatus of the invention, the anchoring body is configured to have a shape adapted to the inner wall of the apex cordis to be attached to the inner wall of the apex cordis; the tissue of the inner wall of the apex cordis is thicker, so that there is no horizontal force as compared to being anchored to a sidewall; therefore, compared to being anchored to other positions of the left ventricle, the anchoring structure and the anchoring way of the invention are not easy to twist the stent body and not easy to cause tear damages to the tissue; and, compared to being released at the sidewall, a linear path is between the anchoring portion of the invention and the stent body, so that the releasing way is relatively easy and will not be disturbed by the chordae tendineae and the papillary muscle.
Fourth, according to the transseptal mitral valve apparatus of the invention, when the anchoring portion is configured to be of an inverted cone structure, a cavity is formed between the anchoring portion and the ventricle wall after the ventricle anchoring member is released, and the cavity reduces a volume of the left ventricle so that a tension of the left ventricle of a patient is reduced, thereby playing a role in treating heart failure; the larger the left ventricle is, the better the therapeutic effect caused by reducing the volume of the left ventricle with the cavity is for the patient with poorer cardiac contractility.

Certainly, any one product for implementing the present invention is unnecessary to achieve all the above advantages at the same time.

### BRIEF DESCRIPTION OF THE DRAWINGS

Fig. 1 is an integral structural diagram of a transseptal mitral valve apparatus according to Embodiment 1 of the invention;
Fig. 2 is an integral structural diagram of an anchoring portion according to Embodiment 1 of the invention;
Fig. 3 is an integral structural diagram of another anchoring portion according to Embodiment 1 of the invention;
Fig. 4 is a partial cross section diagram of a valve prosthesis according to Embodiment 1 of the invention;
Fig. 5 is a diagram of an initial phase of implanting the transseptal mitral valve apparatus according to Embodiment 1 of the invention;
Fig. 6 is a diagram of a release phase of the transseptal mitral valve apparatus according to Embodiment 1 of the invention;
Fig. 7 is a structural diagram of a complete release phase of the transseptal mitral valve apparatus according to Embodiment 1 of the invention;
Fig. 8 is a structural diagram of completing implanting the transseptal mitral valve apparatus according to Embodiment 1 of the invention;
Fig. 9 is an integral structural diagram of a transseptal mitral valve apparatus according to Embodiment 2 of the invention;
Fig. 10 is an integral structural diagram of the anchoring portion according to Embodiment 2 of the invention.

Reference for numerals: stent body 110; first region 101; second region 102; third region 103; skirt 120; prosthetic valve leaflet 130; ventricle anchoring member 140; connecting portion 141; anchoring portion 142; anchoring body 1421; anchoring apparatus 1422; retention portion 1423; end nod A; extension portion B; delivery conduit 201; cavity 301; elastic buffer portion 1424; force dissipating portion 1426; skirt 1427.

### DESCRIPTION OF THE PREFERRED EMBODIMENTS

In the description of the present invention, it should be noted that orientations or position relationships indicated by terms "center", "upper", "lower", "left", "right", "vertical", "horizontal", "inside", "outside" and the like are orientations or position relationships shown in the drawings, and these terms are merely for facilitating description of the present invention and simplifying the description, but not for indicating or implying that the mentioned apparatus or elements must have a specific orientation and must be established and operated in a specific orientation, and thus, these terms cannot be understood as a limitation to the present invention. Moreover, terms like "first", "second", "third" etc. are only used for description, not be considered as a designation or designation of relative importance.

In the description of the present invention, it should be noted that, unless otherwise clearly specified and limited, meanings of terms "install", "connected with", and "connected to" should be understood in a broad sense. For example, the connection may be a fixed connection, a removable connection, or an integral connection; may be a mechanical connection or an electrical connection; may be a direct connection or an indirect connection by using an intermediate medium; or may be intercommunication between two components. For those of ordinary skill in the art, specific meanings of the above terms in the present invention may be understood based on specific situations.

As used in the specification, singular forms "a/an," "one," and "the/that" include plural objects, unless otherwise explicitly stated. As used in the specification, the term "or" is usually used to include the meaning of "and/or", unless otherwise expressly stated.

The following further describes the present invention in combination with specific embodiments.

### Embodiment 1

The embodiment provides a transseptal mitral valve apparatus. The valve apparatus includes a valve prosthesis and a ventricle anchoring member 140, the valve prosthesis includes a stent body 110 playing a supporting role and a prosthetic valve leaflet 130, and the prosthetic valve leaflet 130 is fixed on the stent body 110. The ventricle anchoring member 140 includes a connecting portion 141 and an anchoring portion 142 attached to a ventricle inner all, the anchoring portion 142 being fixed to the stent body 110 by the connecting portion 141. After the prosthesis apparatus is released, the valve prosthesis is fixed at a native valve annulus for replacing a native valve to play a role in opening or closing a blood channel. The anchoring portion 142 provides a firm anchoring force to the stent body 110 so that the stent body 110 is prevented from being impacted into a left atrium.

In the prior art, in order to solve the problem of anchoring the valve prosthesis, normally a way of clamping the valve leaflets or capturing the valve leaflets is used; however, since the native valve annulus and the valve leaflets are of weak structures, this anchoring way inevitably causes damage to the native structure of the valve leaflets. Also, a way of Oversize is used for anchoring, but this way may easily cause the problem of blocking the left ventricle outflow tract. The embodiment changes the traditional anchoring way of clamping the valve leaflets or capturing the valve leaflets, wherein the anchoring portion is released and then attached to the ventricle inner wall to provide a firm anchoring force, not stretch the chordae tendineae or damage the valve leaflets.

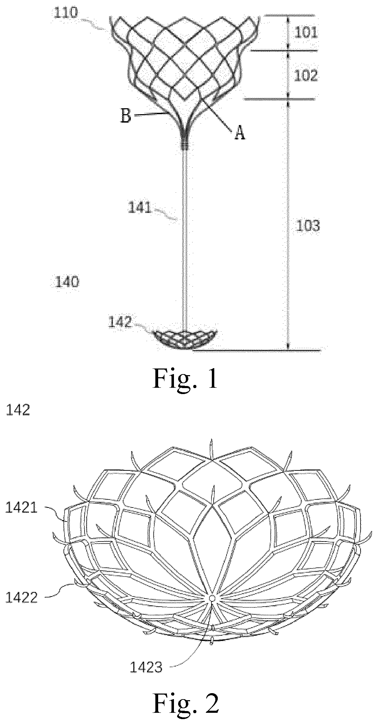

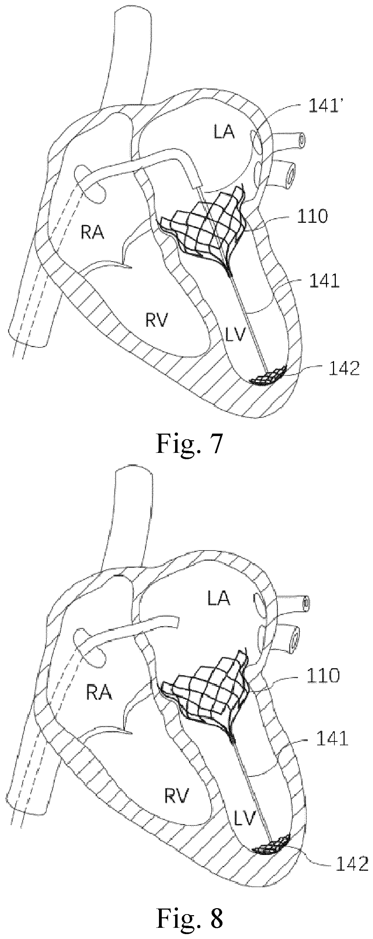

With reference to Figs. 1 to 8, structural diagrams of the transseptal mitral valve apparatus according to the embodiment are shown.

In the embodiment, the anchoring portion 142 includes an anchoring body 1421, and the anchoring body 1421 is configured to be of a mesh structure with a pre-set size and a pre-set shape. The anchoring portion 142 is in a compressed state when being loaded into a delivery system, and then released and expanded to be of the above-mentioned mesh structure with a pre-set size and shape after being implanted into a target position. The mesh structure facilitates enlarging the contact area between the anchoring portion 142 and the tissue to provide more forced points, so that not a single point is forced. Therefore, the forces are dispersed other than concentrated so that the tissue may not be damaged.

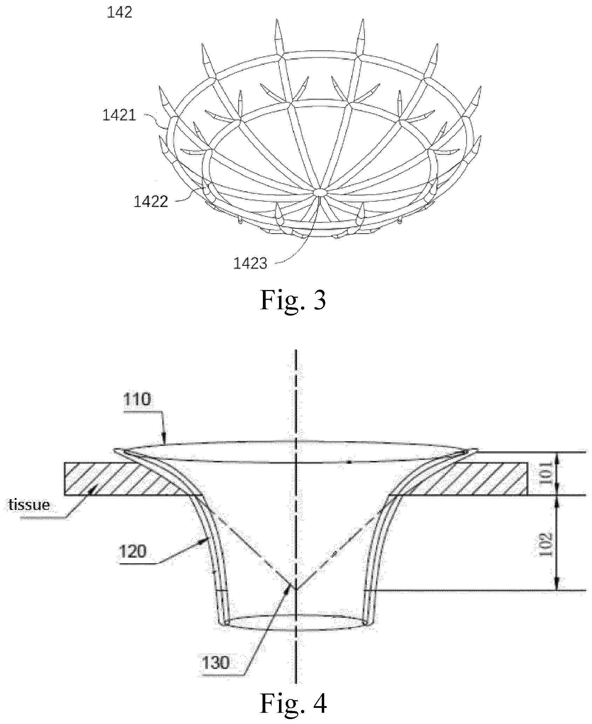

In the embodiment, the mesh structure is formed by arranging several mesh units with an enclosed shape so that it is easier to load and release the anchoring portion 142. The mesh structure formed by arrangement improves the compliance of the anchoring portion 142, and after being released, the anchoring portion 142 is adapted to the shape of the ventricle inner wall for being easier to form an anchoring relationship with the ventricle inner wall. Specifically, the anchoring body 1421 is of the mesh structure formed by a plurality of rows of units, e.g., being formed by arranging mesh units capable of forming the enclosed shape such as a triangle shape, a square shape, a diamond shape, a pentagon shape, a droplet shape or a heart shape. As shown in Fig. 2, the anchoring body 1421 is formed by arranging several mesh units of the diamond shape, and the anchoring body 1421 shown in Fig. 3 is formed by the mesh units of the triangle shape and the square shape.

In the embodiment, the anchoring body 1421 is configured to be in shape adapted to an inner wall of an apex cordis for being attached to the inner wall of an apex cordis. The tissue of the inner wall of the apex cordis is thicker, so that there is no horizontal force as compared to being anchored to a sidewall. Therefore, it is not easy to twist the stent body 110, so it is not easy to cause tear damages to the tissue when the anchoring portion 142 is anchored to the tissue. And, as compared to being released at a sidewall, the region of the apex cordis has no chordae tendineae and papillary muscle and is relatively empty, so being anchored in the region of the apex cordis will not be disturbed by the chordae tendineae and the papillary muscle. In addition, the release path for a situation of being anchored to the inner wall of the apex cordis is a linear path, and the releasing way is relatively easy.

In the embodiment, the anchoring portion 142 has a smaller elastic modulus; after the anchoring portion 142 is released, the anchoring portion 142 has a better compliance to be adapted to different shapes of the apex cordis, so as to be well attached to the inner wall of the apex cordis. Naturally, in some embodiments, the anchoring portion 142 may also be configured to have a smaller stiffness, so that the anchoring portion 142 is softer after being formed to acquire a certain compliance.

Further, with continued reference to Figs. 2 and 3, the anchoring portion 142 further includes several anchoring apparatuses 1422, the anchoring apparatuses 1422 are disposed on the anchoring body 1421, and an enhanced anchoring effect is provided by the fixation between the anchoring apparatus 1422 and a tissue of a ventricle inner wall. In the embodiment, the anchoring apparatus 1422 is an anchoring needle, and the anchoring body 1421 of the mesh structure is directly adhered to the inner wall of the apex cordis, wherein the anchoring needle is disposed at a node of the mesh structure of the anchoring portion 142; the anchoring needle may be located at any one node or all nodes, and the anchoring needle 1422 is inserted into the inner wall of the apex cordis to be anchored together with the tissue at the apex cordis after being implanted. Optionally, the anchoring needle 1422 may be of a barb structure or a clip structure, and establish a firm connection with the tissue at the apex cordis.

In the embodiment, a pre-set position of the anchoring portion 142 is provided with a retention portion 1423, and the connecting portion 141 is connected with the anchoring portion 142 at the retention portion 1423. Specifically, in the embodiment, the anchoring portion 142 is of a disc-shaped structure, the retention portion 1423 is a retention hole located at a center of the anchoring portion 142, and the provision of the retention hole facilitates the stable connection between the connecting portion 141 and the anchoring portion 142. An end portion of the stent body 110 has several end nodes A, wherein the end nodes A extend to form an extension portion B, and the extension portion B is disposed every other end nodes in the several end nodes A; several extension portions B are connected with the other end of the connecting portion 141 at their free ends, thereby fixing the anchoring portion 142 to the stent body 110, as shown in Fig. 1.

In the embodiment, the anchoring portion 142 is made by way of a weaving process or a cutting process. The anchoring portion 142 is further covered with a skirt 120, and the skirt 120 may provide a buffering force while promoting endothelialization in the long run to make the anchoring more stable. The anchoring portion 142 may be selectively made from an elastic or malleable deformable material such as a balloon-expandable material, or may be a shape memory alloy that may respond to temperature changes to convert between a compressed delivery state and an expanded expansion state. The material of the anchoring portion may be the same as or different from that of the stent body.

In the embodiment, the connecting portion 141 may be a tether to provide a traction force to the stent body, so as to prevent the valve prosthesis from being displaced to the left ventricle due to the impact exerted by the blood when the heart contracts. The connecting portion 141 may be made from such as a biocompatible polymer material, which includes, but is not limited to ultra-high molecular weight polyethylene (UHMWPE), polytetrafluoroethylene, and so on. The connecting portion 141 may be elastic-free, so as to provide a more firm anchoring force to the stent. The connecting portion 142 may also be elastic, so that a higher degree of stretch compliance is provided during the cardiac cycle. Optionally, the connecting portion 141 may be made from a bioabsorbable material, and thus provides temporary fixation until the endothelialization between the prosthesis and the tissue is enough to provide the anchoring force of the valve prosthesis.

Specifically, with reference to Figs. 1, 7, and 8, the stent body 110 may provide several functions to the valve prosthesis, which include being used as a body structure of the valve prosthesis, carrying an internal prosthetic valve leaflet 130, being used as a seal for inhibiting the circumferential leakage of valve between the valve prosthesis and the native valve, and being used as a connecting structure (e.g., a hanging tab or a fixing tab) with the delivery system and so on; the delivery system of the embodiment includes a delivery conduit 201. The stent body 110 is of the mesh-shaped structure formed by a plurality of rows of units, e.g. being formed by arranging mesh units capable of forming the enclosed shape such as a triangle shape, a diamond shape, a pentagon shape, a droplet shape, and a heart shape. The stent body 110 may self-expand into a pre-set structure after being released. A material of the stent body 110 may be selectively made from an elastic or malleable deformable material such as a balloon-expandable material, or may be a shape memory alloy that may respond to temperature changes to convert between a compressed delivery state and an inflated expansion state. Specifically, the material may be such as nitinol, titanium alloy, cobalt-chromium alloy, MP35n, 316 stainless steel (SUS316), L605, Phynox/Elgiloy, platinum chromium, or other biocompatible metals known by those skilled in the art. The stent body may be manufactured by a weaving process or a cutting process.

The stent body 110 may be divided into a first region 101, a second region 102, and a third region 103 longitudinally. In the embodiment, after the valve apparatus is implanted into a human body, the first region 101 is located in the ventricle and adhered to a native mitral valve annulus of the heart, so as to prevent the prosthesis valve from falling from the left ventricle to the left atrium. Specifically, the structure for fixing the first region 101 with the valve annulus may select a flange structure, and as shown in Fig. 4, the flange structure is adhered to the valve annulus and located in the ventricle, thereby fixing the stent body 110. The second region 102 is configured to carry the prosthetic valve leaflet 130 while playing a certain role in fixing and sealing by being supported on the tissue; the third region 103 is located in the ventricle, and is an anchoring part of the valve prosthesis in the left ventricle to prevent the prosthesis from being impacted to the left ventricle by the blood when closing. The stent body 110 is further covered with a skirt 120, which plays a sealing role, and the skirt 120 may be of a single-layered or double-layered structure.

The number of the prosthetic valve leaflet 130 may be the same as or different from that of the native valve leaflets, and the prosthetic valve leaflet is fixed to a pre-set position of the second region 102 of the stent body 110 by way of stitching and so on. The prosthetic valve leaflet 130 dynamically switch between the two states of an opening state and a closing state. In some embodiments, the prosthetic valve leaflet 130 may be prepared by biological tissues such as chemically stable tissues from the heart valves of animals such as pigs or pericardium tissues such as cattle (bovine pericardium tissues), sheep (ovine pericardium tissues), pigs (porcine pericardium tissues), and horses (equine pericardium tissues), preferably the bovine pericardium tissues. The prosthetic valve leaflet 130 may also be made from small intestinal submucosa tissues. Further, a synthetic material may also be used for the prosthetic valve leaflet 130, for example, expanded PTFE or polyester. Optionally, the material further includes thermoplastic polyurethane polyester, polyether urethane, segmented polyether urethane, silicone polyether urethane, silicone-polyurethane polyester, and ultra-high molecular weight polyethylene. Moreover, the biocompatible polymer may also be included, which optionally may include polyolefin, elastomer, polyethylene glycol, polyethersulfone, polysulfone, polyvinylpyrrolidone, polyvinyl chloride, other fluorine-containing polymers, silicone polyester, siloxane polymers and/or olimers, and/or polycaprolactone, and block copolymers thereof. Optionally, the prosthetic valve leaflet 130 has a surface treated by (or reacted with) anticoagulants, and the anticoagulant includes but is not limited to heparinized polymers.

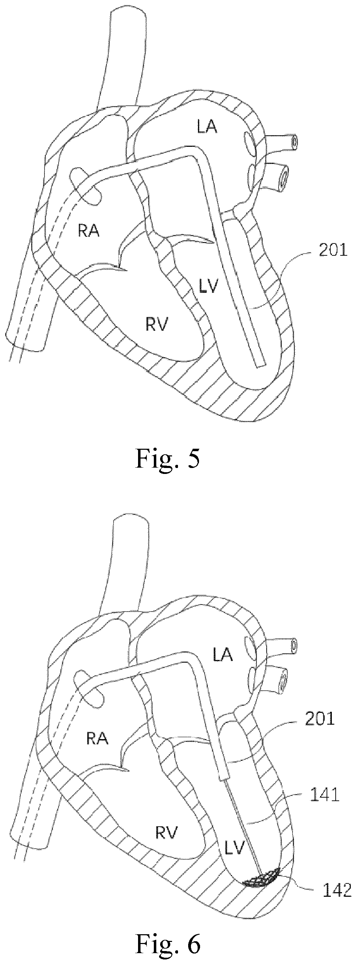

Figs. 5 to 8 describe an implanting process of the valve prosthesis, wherein the delivery conduit 201 enters the right atrium via the inferior vena cava and then passes the interatrial septum and the mitral valve to reach near the apex cordis. When the valve prosthesis is released, the anchoring portion 142 is first released to the apex cordis, and the anchoring apparatus 1422 on the anchoring portion 142 is connected with the inner wall of the apex cordis for anchoring with each other after being released out of the delivery system. Subsequently, the connecting portion 141 and the stent body 110 are continuously released out. After the stent body 110 is completely released out, a tension of the connecting portion 141 is adjusted to reach an optimal anchoring and sealing effect, and the implanting effect of the valve should be confirmed to ensure that there are no problems such as circumferential valve leakage. Then, the connecting portion 141 is fixed and cut, followed by withdrawing the delivery catheter to complete the release.

The connection according to this embodiment, e.g., the connection between the connecting portion 141 and the anchoring portion 142, the connection between the prosthetic valve leaflet 130 and the stent body, and so on, may be performed by way of such as riveting, welding, buckling, stitching or adhering with an adhesive, which may be selected according to actual needs.

### Embodiment 2

The embodiment provides a transseptal mitral valve apparatus, which is an improvement based on Embodiment 1, wherein in the embodiment, the anchoring portion 142 is configured to be of an inverted cone structure, so that a cavity 301 is formed between the anchoring portion 142 and the ventricle wall to reduce a volume of a left ventricle after the ventricle anchoring member is released.

The left ventricle of the patient with dilated cardiomyopathy enlarges, leading to a disproportionate increase in the tension of the left ventricle and an increase in oxygen consumption, which reduces the efficiency of the left ventricle. In the embodiment, the volume of the left ventricle is reduced by the cavity 301 formed between the anchoring portion and the ventricle wall after the anchoring portion is released, so that a tension of the left ventricle of a patient is reduced, thereby playing a role in treating heart failure. The larger the left ventricle is, the better the therapeutic effect caused by reducing the volume of the left ventricle with the cavity 301 is for the patient with poorer cardiac contractility.

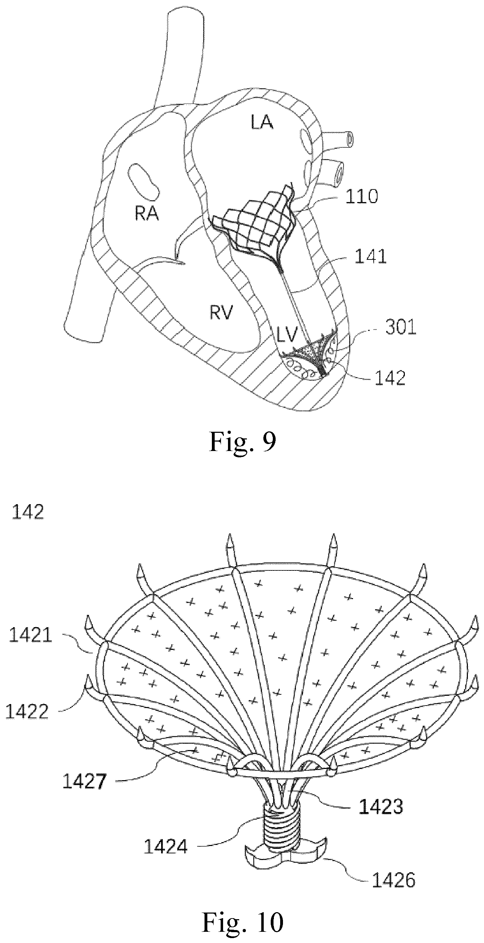

With reference to Figs. 9 to 10, structural diagrams of the transseptal mitral valve apparatus according to the embodiment are shown.

Specifically, in the embodiment, an end portion of the anchoring portion 142 away from the stent body 110 is provided with a force dissipating portion 1426 for increasing a contact area, wherein the force dissipating portion 1426 may be configured to be of a laminar structure, so as to enlarge the contact area with the tissue. Further, an elastic buffer portion 1424 is disposed between the force dissipating portion 1426 and the end portion of the anchoring portion 142, and the elastic buffer portion 1424 is configured to be of a spiral-shaped structure or a spring-shaped structure to provide a buffering effect. The force dissipating portion 1426 and the elastic buffer portion 1424 prevent the inner wall of the apex cordis from being damaged by the anchoring portion 142 when the heart contracts.

In the embodiment, the anchoring apparatus 1422 is disposed on the anchoring portion 142 and on a position contacting the tissue such as on a top end of a frame body 1421 of the anchoring portion 142 or on a force dissipating apparatus 1426; the anchoring portion 142 is anchored with the inner wall of the apex cordis by the anchoring apparatus 1422. This anchoring structure causes the valve apparatus of the embodiment to be implanted from the interatrial septum without thoracotomy, which causes less damage on the patient as compared to being implanted from the apex cordis.

In the embodiment, the anchoring portion is covered with a skirt 120 that is used to be cooperated with the ventricle wall to form the cavity. Specifically, the skirt 120 may be of the single-layered structure, or may be of an internal-external double-layered structure, which may select materials such as PTFE, ePTFE, and polyester fabrics made by knitting, tatting, or weaving, so as to mainly play a sealing role for isolating the cavity 301.

According to the valve apparatus of the embodiment, the inverted cone structure of the anchoring portion causes the above cavity to be isolated between the structure and the ventricle inner wall after the structure is released, thereby treating the ventricular enlargement. In other words, the valve apparatus of the embodiment may be configured to replace the native valve while having the function of reducing the volume of the ventricle and improving the functions of the heart, thereby showing an important significance clinically.

In the invention, after the valve apparatus is released, the valve prosthesis is fixed at a native valve annulus for replacing a native valve to play a role in opening or closing a blood flow channel, and the ventricle anchoring member provides a firm anchoring force for the stent body by the attachment between the anchoring portion and the ventricle wall so that the stent body is prevented from being impacted into a left atrium.

The above disclosure is only the preferred embodiment of the present invention. The preferred embodiments do not describe all the details, and are not intended to limit the invention only to be the specific embodiments. It should be understood that these embodiments are only used for illustrating the present invention, but not for the limitation of the scope of the present invention. In practical application, the improvements and adjustments made by those skilled in the art according to the present invention still fall within the scope of protection of the present invention.

It is obvious that various modifications and changes can be made to the content of the specification. The present invention selects and specifically describes the embodiments with the purpose of better explain the principle and practical use of the present invention, such that a person skilled in the art can well utilize the present invention. The present invention is merely limited by the appended claims and the scope and equivalents thereof.

## Claims

1. A transseptal mitral valve apparatus, comprising:
a valve prosthesis, comprising a stent body playing a supporting role and a prosthetic valve leaflet, the prosthetic valve leaflet being fixed to the stent body; and
a ventricle anchoring member, comprising a connecting portion and an anchoring portion attached into a ventricle inner wall, the anchoring portion being fixed to the stent body by the connecting portion;
after the valve apparatus is released, the valve prosthesis is fixed at a native valve annulus for replacing a native valve to play a role in opening or closing a blood flow channel, and the ventricle anchoring member provides a firm anchoring force to the stent body by the attachment between the anchoring portion and the ventricle wall so that the stent body is prevented from being impacted into a left atrium.

2. The transseptal mitral valve apparatus according to claim 1, wherein the anchoring portion comprises an anchoring body, and the anchoring body is configured to be of a mesh structure with a pre-set size and a pre-set shape.

3. The transseptal mitral valve apparatus according to claim 2, wherein the anchoring body is configured to be in a shape suitable for an inner wall of an apex cordis to be attached to the inner wall of an apex cordis.

4. The transseptal mitral valve apparatus according to claim 1, 2, or 3, wherein the anchoring portion further comprises several anchoring apparatuses, the anchoring apparatuses are disposed on the anchoring body, and an enhanced anchoring effect is provided by the fixation between the anchoring apparatus and a tissue of a ventricle inner wall.

5. The transseptal mitral valve apparatus according to claim 1, wherein the anchoring portion is provided with a retention portion, and the connecting portion is connected with the anchoring portion at the retention portion.

6. The transseptal mitral valve apparatus according to claim 1, wherein the connecting portion is elastic-free, or the connecting portion is elastic.

7. The transseptal mitral valve apparatus according to claim 1, wherein the anchoring portion is configured to be of an inverted cone structure, so that a cavity is formed between the anchoring portion and the ventricle wall to reduce a volume of a left ventricle after the ventricle anchoring member is released.

8. The transseptal mitral valve apparatus according to claim 7, wherein an end portion of the anchoring portion away from the stent body is provided with a force dissipating portion for increasing a contact area.

9. The transseptal mitral valve apparatus according to claim 8, wherein an elastic buffer portion is disposed between the force dissipating portion and the end portion of the anchoring portion.

10. The transseptal mitral valve apparatus according to any one of claims 1, 2, 3, 5-9, wherein the anchoring portion is further covered with a skirt.

11. A method for implanting a transseptal mitral valve apparatus, comprising providing a transseptal mitral valve apparatus, wherein the valve apparatus comprises a valve prosthesis and a ventricle anchoring member, the valve prosthesis comprising a stent body playing a supporting role and a prosthetic valve leaflet, the prosthetic valve leaflet being fixed at a pre-set position of the stent body; the ventricle anchoring member comprises a connecting portion and an anchoring portion attached to a ventricle inner all, the anchoring portion being fixed to the stent body by the connecting portion.
